# EUROPÄISCHE PATENTSCHRIFT

(11) **EP 4 326 178 B1**
(45) Veröffentlichungstag und Bekanntmachung des Hinweises auf die Patenterteilung: **19.02.2025**
(21) Anmeldenummer: 21754900.5
(22) Anmeldetag: 10.08.2021
(51) Int. Cl.: A61B 17/80, A61B 17/82

(54) **OLEKRANON-KNOCHENPLATTE**
OLECRANON BONE PLATE
PLAQUE OSSEUSE D'OLÉCRANE

(43) Veröffentlichungstag der Anmeldung: 28.02.2024
(73) Patentinhaber: Bonebridge AG, 6300 Zug (CH)
(72) Erfinder: BURKI, Patrick, 4500 Solothurn (CH); MOOR, Beat Kaspar, 3973 Venthône (CH); SPROSS, Christian, 9422 Staad (CH); OLACH, Martin, 9016 St. Gallen (CH)
(74) Vertreter: Dr. Lusuardi AG
(86) Internationale Anmeldenummer: PCT/CH2021/050016
(87) Internationale Veröffentlichungsnummer: WO 2023/015400

(56) Entgegenhaltungen:
- EP-A1- 3 808 295
- FR-A1- 2 899 094
- US-A1- 2004 116 930
- US-A1- 2015 196 333

## Beschreibung

Die Erfindung bezieht sich auf eine Knochenplatte geeignet für die Versorgung von Frakturen, Non-Unions und für Osteotomien des Olekranons und der proximalen Ulna gemäss dem Oberbegriff des Patentanspruchs 1,

Proximale Ulna/Olekranon-Frakturen sind relativ häufige Verletzungen bei Erwachsenen und können zu schweren Funktionsstörungen führen, die sich aus posttraumatischer Instabilität, Impingement, Weichteilkontrakturen, Malunion oder Nonunion ergeben. Verletzungen mit hoher Energie können zu assoziierten distalen Verletzungen führen, wie z. B. Unterarm- und distale Radiusfrakturen, Frakturen einschließlich der intraossären Membran oder Disruptionen des distalen Radioulnargelenks. Monteggia-Frakturen sind Frakturen des proximalen Drittels der Ulna mit assoziierter Radiuskopf-Dislokation.

Die erfindungsgemässe Knochenplatte ist für die Verwendung bei der offenen Reposition und internen Fixation (ORIF) des Olekranons und der proximalen Ulna vorgesehen. Sie ist indiziert für Frakturen, Osteotomien und Non-Unions des Olekranons und der proximalen Ulna.

Aus der US 8,182,517 ist eine Knochenplatte für die interne Fixation einer Fraktur an der proximalen Ulna bekannt, welche einen schmalen, proximalen Kopfteil aufweist, welcher gegenüber dem breiteren, distalen Schaftteil abgebogen ist und eine oder zwei auf der longitudinalen Zentralachse der Knochenplatte angeordnete Bohrung(en) aufweist.

Diese bekannte Knochenplatte weist jedoch den Nachteil auf, dass der abgebogene Kopfteil relativ zum Schafteil kurz und schmal ausgebildet ist, so dass damit nicht alle intraartikulären Frakturen versorgt werden können. Zudem ist es schwierig die Fragmente bei einem Bruch nur mit einer Schraube (oder mit zwei longitudinal angeordneten Schrauben) passend fixieren zu können.

Eine Knochenplatte mit den Merkmalen des Oberbegriffs des Anspruchs 1 ist aus der EP 3 808 295 A1 bekannt.

Hier will die Erfindung Abhilfe schaffen. Der Erfindung liegt die Aufgabe zugrunde, eine Knochenplatte für das Olekranon zu schaffen, welche es gestattet jede Art von Fraktur zu versorgen.

Die Erfindung löst die gestellte Aufgabe mit einer Knochenplatte, welche die Merkmale gemäss dem Anspruch 1 aufweist; und einer Kombination einer erfindungsgemässen Knochenplatte mit mindestens einer polyaxialen Knochenschraube, welche die Merkmale gemäss dem Anspruch 14 aufweist.

Die durch die Erfindung erreichten Vorteile sind im Wesentlichen darin zu sehen, dass dank der erfindungsgemässen Knochenplatte mit einem verbreiterten Kopfteil, welcher mit drei Schraubenbohrungen ausgestattet ist, ein gebrochenes Olekranon optimal abgestützt werden kann. Durch die zwei links und rechts von der Zentralachse angeordneten Schraubenbohrungen können zwei Schrauben können beinahe parallel zum Schaftteil gesetzt werden, so dass alle Zwischenfragmente optimal gefasst werden können und eine optimale mechanische Stabilität erreicht wird.

Weitere vorteilhafte Ausgestaltungen der Erfindung können wie folgt kommentiert werden:
Bei einer besonderen Ausführungsform der erfindungsgemässen Knochenplatte weist der proximale Kopfteil eine dritte Bohrung mit einer Bohrungsachse auf, welche mittig zur Zentralachse angeordnet ist. Durch die zusätzliche dritte Bohrung lassen sich mit der Knochenplatte praktisch alle möglichen Frakturen versorgen. Beispielsweise lässt sich mit dieser dritten, am proximalsten angeordneten Bohrung auch ein einfacher intraartikulärer Olekranonbruch versorgen. Dabei werden kaum alle drei proximalen Bohrungen mit Knochenschrauben bestückt, weil sonst der Platz zu eng würde, aber je nach Fraktur hat der Operateur die Wahl, wo er eine Schraube im Kopfteil setzen möchte.

Die Länge L_{K} des gegenüber dem Schafteil abgebogenen proximalen Kopfteils beträgt mindestens 20 %, vorzugsweise mindestens 25 % der Länge Ls des Schaftteils. Durch den relativ langen Kopfteil ist es möglich das ganze proximale Olekranon abzustützen.

Bei einer weiteren Ausführungsform ist das freie proximale Ende des Kopfteils gegenüber dem distalen Schafteil mindestens um 70°, vorzugsweise um mindestens 80° abgebogen.

Bei einer speziellen Ausführungsform konvergieren die beiden Bohrungsachsen nach distal und der Winkel a ist grösser als 1°, vorzugsweise grösser als 4°. Dadurch erreicht man, dass die polyaxialen Schrauben, wenn diese in Nullgradrichtung zur Bohrungsachse gesetzt werden, nicht in das Gelenk geschossen werden können.

Bei einer nicht beanspruchten Ausführungsform sind die Zentren sämtlicher Plattenbohrungen im distalen Schaftteil höchstens 2 mm von der Zentralachse entfernt sind.

Bei einer besonderen Ausführungsform weist mindestens eine der Bohrungen oder der Plattenbohrungen eine Innenwandung aus einem Material auf, welches eine Härte H_{P} besitzt. Zudem ist in der Plattenbohrung ein hohlzylinder- oder hohlkegelförmiger Einsatz gelagert, der zumindest teilweise an der Innenwandung anliegt und zur Aufnahme des Kopfes einer Knochenschraube geeignet ist, wobei der Einsatz in der Plattenbohrung verdrehgesichert angeordnet ist und aus einem Material besteht, das eine Härte H_{E} < H_{P} aufweist. Dies ermöglich das Setzen einer Verriegelungsschraube in den kreisrunden Plattenbohrungen.

Bei einer nicht beanspruchten Ausführungsform weist der distale Schaftteil ein Langloch zur Ausübung einer axialen oder interfragmentären Kompression auf.

Bei einer nicht beanspruchten Ausführungsform weist der distale Schaftteil ein Fadenloch mit geringerem Durchmesser im Vergleich zum Durchmesser der Bohrungen auf, welches geeignet ist umliegende Weichteile an die Knochenplatte zu fixieren. Das Fadenloch ist zweckmässigerweise zwischen dem Langloch und dem proximal nächstliegenden Plattenloch angeordnet.

Bei einer besonderen Ausführungsform ist die durchschnittliche Dicke der Knochenplatte in der Übergangszone zwischen dem distalen Schaftteil und dem proximalen Kopfteil kleiner als die durchschnittliche Dicke des distalen Schaftteils, vorzugsweise 10 % kleiner.

Bei einer weiteren Ausführungsform ist mindestens zwischen zwei Plattenbohrungen des distalen Schafteils eine Einschnürung vorgesehen, so dass die Breite vorzugsweise um mindestens 20 % reduziert ist, was das Verbiegen der Knochenplatte durch den Operateur erleichtert. Das Flächenträgheitsmoment des Plattenquerschnitts an der engsten Stelle der Einschnürung entspricht vorteilhafterweise im Wesentlichen dem Flächenträgheitsmoment des Plattenquerschnitts im Bereich des grössten Durchmessers der benachbarten Plattenbohrung. Die Knochenplatte lässt sich damit überall gleich gut biegen und es entstehen unter der Dauerbelastung keine Schwachstellen.

Bei einer weiteren Ausführungsform der erfindungsgemässen Knochenplatte weist der proximale Kopfteil ein Durchgangsloch zur Aufnahme eines Kirschnerdrahts auf, dessen Lochachse vorzugsweise parallel zu einer der Bohrungsachsen der Bohrungen verläuft. Damit kann die Fraktur reponiert und gehalten werden und die optimale Schraubenausrichtung definiert werden.

Vorteilhafterweise ist der distale Schaftteil frei von lateralen, quer zur Zentralachse verlaufenden Flügeln, insbesondere von solchen mit einer Schraubenbohrung.

Bei einer nicht beanspruchten Ausführungsform der erfindungsgemässen Knochenplatte liegt die Länge L_{K} des gegenüber dem Schafteil abgebogenen proximalen Kopfteils im Bereich von 16 - 24 mm, vorzugsweise im Bereich von 17 - 23 mm.

Die erfindungsgemässe Knochenplatte kann auch spiegelverkehrt ausgeführt werden.

Die erfindungsgemässe Knochenplatte kann sowohl als solche zur Verfügung gestellt werden als auch in Kombination mit mindestens einer polyaxialen Knochenschraube, z.B. in Form eines sterilen Kits.

Die erfindungsgemässe Knochenplatte eignet sich besonders gut zur Versorgung von Frakturen, Non-Unions und für Osteotomien des Olekranons und der proximalen Ulna.

Die Erfindung und Weiterbildungen der Erfindung werden im Folgenden anhand der teilweise schematischen Darstellungen eines Ausführungsbeispiels noch näher erläutert.

Es zeigen:
Fig. 1 eine seitliche perspektivische Ansicht einer Ausführungsform der erfindungsgemässen Knochenplatte;
Fig. 2 eine frontale perspektivische Ansicht der in Fig. 1 dargestellten Ausführungsform;
Fig. 3 eine weitere perspektivische Ansicht von oben der in Fig. 1 dargestellten Ausführungsform; und
Fig. 4 eine Seitenansicht der in Fig. 1 dargestellten Ausführungsform,

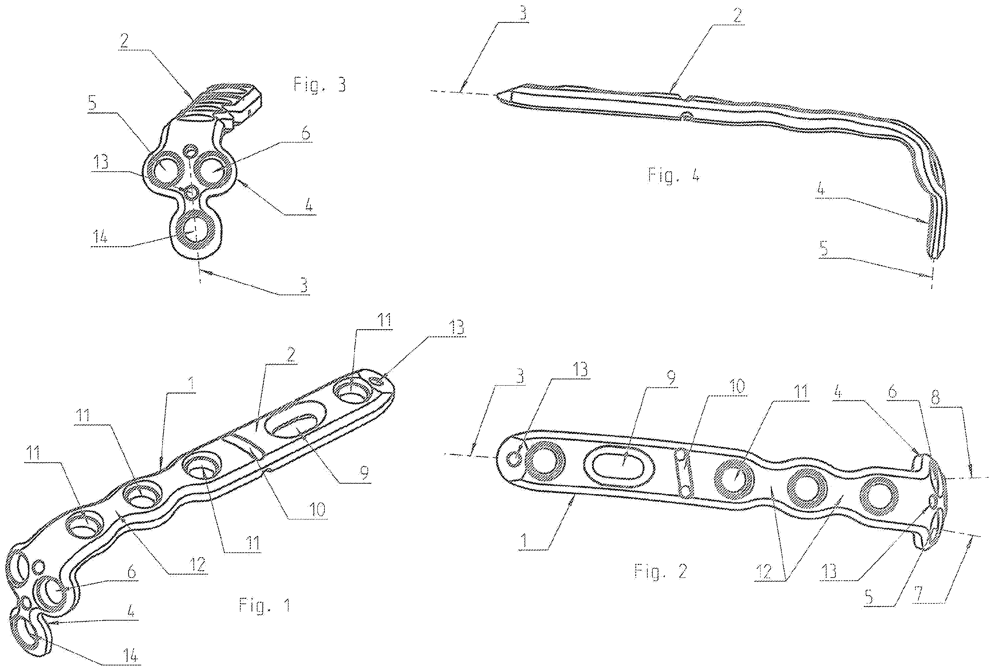

Die in den Fig. 1 bis 4 dargestellte Ausführungsform zeigt eine Knochenplatte 1 zur Versorgung von Frakturen, Non-Unions und für Osteotomien des Olekranons und der proximalen Ulna.

Wie in Fig.1 dargestellt besteht die Knochenplatte 1 aus einem distalen longitudinalen Schaftteil 2 und einem daran anschliessenden proximalen Kopfteil 4. Die Länge des gegenüber dem Schaftteil 2 abgebogen proximalen Kopfteils 4 beträgt ca. 20 mm, was ca. 30 % der Länge des Schaftteils 2 entspricht.

Der distalen Schaftteil 2 weist vier, längs der Zentralachse 3 angeordnete Plattenbohrungen 11 und eine Langloch 9 auf. Die Knochenplatte 1 weist eine longitudinale Zentralachse 3 auf.

Die Zentren sämtlicher Plattenbohrungen 11 und des Langlochs 9 im distalen Schaftteil 2 befinden sich auf der Zentralachse 3. Der distale Schaftteil 2 weist zudem ein Fadenloch 10 aufweist, zur Aufnahme eines Fadens, mit welchem umliegende Weichteile an die Knochenplatte 1 fixiert werden können.

Das Fadenloch 10 ist zwischen dem Langloch 9 und dem proximal nächstliegenden Plattenloch 11 angeordnet. Das Langloch 9 liegt zwischen den drei proximal davon angeordneten und der distal davon angeordnete vierten Plattenbohrung 11. Der distalste Bereich des Schaftteils 2 ist abgeschrägt und weist ein Durchgangsloch 13 zur Aufnahme eines Kirschnerdrahts auf.

Zwischen zwei Plattenbohrungen 11 des distalen Schafteils 2, der eine Breite von 10,4 mm aufweist befindet sich jeweils eine Einschnürung 12, welche die Schaftbreite in diesem Bereich auf 7,5 mm reduziert.

Der Kopfteil 4 ist gegenüber dem distalen Schaftteil 2 abgebogen. Aus Fig. 4 ist ersichtlich, dass der proximalste Bereich des Kopfteils 4 einen Winkel von annähernd 90° zum Schaftteil 2 aufweist. Der Kopfteil 4 ist zudem gegenüber dem Schafteil 2 verbreitert.

Die Dicke der Knochenplatte 1 in der Übergangszone zwischen dem distalen Schaftteil 2 und dem proximalen Kopfteil 4 ist kleiner als die durchschnittliche Dicke des distalen Schaftteils 2 von 3,2 mm und variiert zwischen 2,5 - 2,8 mm.

Der proximale Kopfteil 4 besitzt insgesamt drei Bohrungen 5,6 und 14 wobei zwei der drei Bohrungen 5,6 auf gegenüberliegenden Seiten der Zentralachse 3 angeordnet sind und die dritte Bohrung 14 auf der Zentralachse 3 angeordnet ist. Die auf gegenüberliegenden Seiten der Zentralachse 3 liegenden Bohrungen 5,6 besitzen gegen distal hin konvergierende Bohrungsachsen 7,8, welche einen Winkel α von 7° einschliessen. Die beiden Bohrungen 5,6 sind derart ausgebildet, dass sie sowohl geeignet sind (i) polyaxiale Verrieglungsschrauben unter einem Winkel von ±15° gegenüber ihren jeweiligen Bohrungsachse 7;8, als auch (ii) nicht verriegelbare Standardschraube aufzunehmen.

Der Kopfteil 4 besitzt zudem zwei Durchgangslöcher 13 zur Aufnahme eines Kirschnerdrahts; das eine Durchgangsloch 13 ist distal zur zentral angeordneten dritten Bohrung 14 angeordnet und das zweite Durchgangsloch 13 liegt distal zwischen den beiden rechts und links der Zentralachse 3 angeordneten Bohrungen 5,6.

Als Variante zur Ausführungsform gemäss den Figuren 1 - 4 kann der distale Schaftteil 2 statt vier Plattenbohrungen 11 deren acht aufweisen, drei davon proximal des Langlochs 9 und die restlichen fünf distal des Langlochs 9 angeordnet. Bei dieser Knochenplatte mit einem verlängerten Schaftteil 2 beträgt die Länge des gegenüber dem Schaftteil 2 abgebogenen proximalen Kopfteils 4 ca. 15 % der Länge des Schaftteils 2.

## Patentansprüche

1. Knochenplatte (1) geeignet für die Versorgung von Frakturen, Non-Unions und für Osteotomien des Olekranons und der proximalen Ulna, mit
A) einem distalen longitudinalen Schaftteil (2), welcher einer Anzahl von Plattenbohrungen (11) aufweist;
B) einer longitudinalen Zentralachse (3);
C) einem proximalen Kopfteil (4), welcher gegenüber dem distalen Schaftteil verbreitert ist und zwei auf gegenüberliegenden Seiten der Zentralachse (3) angeordnete Bohrungen (5;6) mit den Bohrungsachsen (7,8) aufweist; wobei
D) der Kopfteil (4) gegenüber dem Schafteil (2) abgebogen ist; und
E) die beiden Bohrungen (5,6) zur Aufnahme
(i) einer polyaxialen Verrieglungsschraube unter einem Winkel von ±15° gegenüber der jeweiligen Bohrungsachse (7;8); oder
(ii) einer nicht verriegelbaren Standardschraube geeignet sind;
**dadurch gekennzeichnet, dass**
F) die beiden Bohrungsachsen (7,8) nach distal konvergieren und einen Winkel α grösser als 1° einschliessen.

2. Knochenplatte (1) nach Anspruch 1, **dadurch gekennzeichnet, dass** der proximale Kopfteil (4) eine dritte Bohrung (14) mit der Bohrungsachse (15) aufweist, welche zentrisch zur Zentralachse (3) angeordnet ist.

3. Knochenplatte (1) nach Anspruch 1 oder 2, **dadurch gekennzeichnet, dass** die Länge L_{K} des gegenüber dem Schafteil (2) abgebogenen proximalen Kopfteils (4) mindestens 20 %, vorzugsweise mindestens 25 % der Länge L_{S} des Schaftteils (2) beträgt.

4. Knochenplatte (1) nach einem der Ansprüche 1 bis 3, **dadurch gekennzeichnet, dass** das freie proximale Ende des Kopfteils (4) gegenüber dem distalen Schafteil (2) mindestens um 70°, vorzugsweise um mindestens 80° abgebogen ist.

5. Knochenplatte (1) nach einem der Ansprüche 1 bis 4, **dadurch gekennzeichnet, dass** der Winkel α grösser als 4° ist.

6. Knochenplatte (1) nach einem der Ansprüche 1 bis 5, **dadurch gekennzeichnet, dass** mindesten eine der Bohrungen (5,6) oder der Plattenbohrungen (11) eine Innenwandung aus einem Material aufweist, das eine Härte H_{P} besitzt, und dass in der Plattenbohrung ein hohlzylinder- oder hohlkegelförmiger Einsatz gelagert ist, der zumindest teilweise an der Innenwandung anliegt und zur Aufnahme des Kopfes einer Knochenschraube geeignet ist, wobei der Einsatz in der Plattenbohrung verdrehgesichert angeordnet ist und aus einem Material besteht, das eine Härte H_{E} < H_{P} aufweist.

7. Knochenplatte (1) nach einem der Ansprüche 1 bis 6, **dadurch gekennzeichnet, dass** der distale Schaftteil (2) ein Fadenloch (10) mit geringerem Durchmesser im Vergleich zum Durchmesser der Bohrungen (5,6) aufweist und welches geeignet ist umliegende Weichteile an die Knochenplatte (1) zu fixieren.

8. Knochenplatte (1) nach einem der Ansprüche 1 bis 7, **dadurch gekennzeichnet, dass** die durchschnittliche Dicke der Knochenplatte (1) in der Übergangszone zwischen dem distalen Schaftteil (2) und dem proximalen Kopfteil (4) kleiner ist als die durchschnittliche Dicke des distalen Schaftteils (2), vorzugsweise 10 % kleiner.

9. Knochenplatte (1) nach einem der Ansprüche 1 bis 8, **dadurch gekennzeichnet, dass** mindestens zwischen zwei Plattenbohrungen (11) des distalen Schafteils (2) eine Einschnürung (12) vorgesehen ist, so dass die Breite des distalen Schafteils (2) vorzugsweise um mindestens 20 % reduziert ist.

10. Knochenplatte (1) nach einem der Ansprüche 1 bis 9, **dadurch gekennzeichnet, dass** der proximale Kopfteil (4) ein Durchgangsloch (13) zur Aufnahme eines Kirschnerdrahts aufweist, dessen Lochachse vorzugsweise parallel zu einer der Bohrungsachsen (7,8,15) der Bohrungen (5,6,14) verläuft.

11. Knochenplatte (1) nach einem der Ansprüche 1 bis 10, **dadurch gekennzeichnet, dass** der distale Schaftteil (2) frei von lateralen, quer zur Zentralachse (3) verlaufenden Flügeln ist, insbesondere von solchen mit einer Schraubenbohrung.

12. Knochenplatte (1) nach einem der Ansprüche 1 bis 11, **dadurch gekennzeichnet, dass** ihre Geometrie an die Anatomie der rechtsseitigen Ulna (1) angepasst ist.

13. Knochenplatte (1) nach Anspruch 12, **dadurch gekennzeichnet, dass** ihre Geometrie spiegelverkehrt zu dieser an die linksseitige Ulna (1) angepasst ist.

14. Kombination einer Knochenplatte (1) gemäss einem der Ansprüche 1 bis 13 und mindestens einer polyaxialen Knochenschraube.

15. Knochenplatte (1) gemäss einem der Ansprüche 1 bis 13, geeignet zur Behandlung von Frakturen und Non-Unions des Olekranons und der proximalen Ulna sowie dessen Osteotomien.

## Claims

1. Bone plate (1) suitable for the treatment of fractures, non-unions and for osteotomies of the olecranon and the proximal ulna, with
A) a distal longitudinal shaft part (2), which has a number of plate holes (11);
B) a longitudinal central axis (3);
C) a proximal head part (4), which is widened relative to the distal shaft part and has two bores (5; 6) arranged on opposite sides of the central axis (3) with the bore axes (7, 8); wherein
D) the head part (4) is bent with respect to the shaft part (2); and
E) the two bores (5, 6) are suitable for receiving
(i) a polyaxial locking screw at an angle of ±15° relative to the respective bore axis (7;8); or
(ii) a non-locking standard screw;
**characterized in that**
F) the two bore axes (7, 8) converge distally and include an angle α greater than 1°.

2. Bone plate (1) according to claim 1, **characterized in that** the proximal head part (4) has a third bore (14) with the bore axis (15), which is arranged centrally to the central axis (3).

3. Bone plate (1) according to claim 1 or 2, **characterized in that** the length LK of the proximal head part (4), which is bent with respect to the shaft part (2), is at least 20%, preferably at least 25%, of the length LS of the shaft part (2).

4. Bone plate (1) according to one of claims 1 to 3, **characterized in that** the free proximal end of the head part (4) is bent by at least 70°, preferably by at least 80°, with respect to the distal shaft part (2).

5. Bone plate (1) according to one of claims 1 to 4, **characterized in that** the angle α is greater than 4°.

6. Bone plate (1) according to one of claims 1 to 5, **characterized in that** at least one of the bores (5, 6) or the plate bores (11) has an inner wall made of a material which has a hardness HP, and **in that** a hollow cylindrical or hollow conical insert is mounted in the plate bore, which insert at least partially abuts against the inner wall and is suitable for receiving the head of a bone screw, the insert being arranged in the plate bore in a non-rotatable manner and consisting of a material which has a hardness HE < HP.

7. Bone plate (1) according to one of claims 1 to 6, **characterized in that** the distal shaft part (2) has a thread hole (10) with a smaller diameter compared to the diameter of the bores (5, 6) and which is suitable for fixing surrounding soft parts to the bone plate (1).

8. Bone plate (1) according to one of claims 1 to 7, **characterized in that** the average thickness of the bone plate (1) in the transition zone between the distal shaft part (2) and the proximal head part (4) is smaller than the average thickness of the distal shaft part (2), preferably 10% smaller.

9. Bone plate (1) according to one of claims 1 to 8, **characterized in that** a constriction (12) is provided at least between two plate bores (11) of the distal shaft part (2), so that the width of the distal shaft part (2) is preferably reduced by at least 20%.

10. Bone plate (1) according to one of claims 1 to 9, **characterized in that** the proximal head part (4) has a through hole (13) for receiving a Kirschner wire, the hole axis of which preferably runs parallel to one of the bore axes (7, 8, 15) of the bores (5, 6, 14).

11. Bone plate (1) according to one of claims 1 to 10, **characterized in that** the distal shaft part (2) is free from lateral wings extending transversely to the central axis (3), in particular from wings with a screw hole.

12. Bone plate (1) according to one of claims 1 to 11, **characterized in that** its geometry is adapted to the anatomy of the right-sided ulna (1).

13. Bone plate (1) according to claim 12, **characterized in that** its geometry is adapted to the left-sided ulna (1) in a mirror-inverted manner.

14. Combination of a bone plate (1) according to any one of claims 1 to 13 and at least one polyaxial bone screw.

15. Bone plate (1) according to any one of claims 1 to 13, suitable for the treatment of fractures and non-unions of the olecranon and the proximal ulna as well as osteotomies thereof.

## Revendications

1. Plaque osseuse (1) appropriée pour le traitement de fractures, de non-unions et pour des ostéotomies de l'olécrane et du cubitus proximal, comprenant
A) une partie de tige longitudinale distale (2), qui présente un certain nombre de trous de plaque (11) ;
B) un axe longitudinal central (3) ;
C) une partie de tête proximale (4) qui est élargie par rapport à la partie de tige distale et qui présente deux alésages (5 ; 6) disposés sur des côtés opposés de l'axe central (3) avec les axes d'alésage (7, 8) ; où
D) la partie de tête (4) est courbée par rapport à la partie de tige (2) ; et
E) les deux alésages (5, 6) sont adaptés à recevoir
(i) une vis de verrouillage polyaxiale sous un angle de ±15° par rapport à l'axe de l'alésage respectif (7 ; 8) ; ou
(ii) une vis standard non verrouillable ;
**caractérisé en ce que**
F) les deux axes de perçage (7, 8) convergent vers le côté distal et forment un angle α supérieur à 1 °.

2. Plaque osseuse (1) selon la revendication 1, **caractérisée en ce que** la partie de tête proximale (4) présente un troisième alésage (14) avec l'axe de l'alésage (15) qui est centré par rapport à l'axe central (3).

3. Plaque osseuse (1) selon la revendication 1 ou 2, **caractérisée en ce que** la longueur LK de la partie de tête proximale (4) pliée par rapport à la partie de tige (2) représente au moins 20 %, de préférence au moins 25 % de la longueur LS de la partie de tige (2).

4. Plaque osseuse (1) selon l'une des revendications 1 à 3, **caractérisée en ce que** l'extrémité proximale libre de la partie de tête (4) est courbée d'au moins 70°, de préférence d'au moins 80°, par rapport à la partie de tige distale (2).

5. Plaque osseuse (1) selon l'une des revendications 1 à 4, **caractérisée en ce que** l'angle α est supérieur à 4°.

6. Plaque osseuse (1) selon l'une des revendications 1 à 5, **caractérisée en ce qu'**au moins l'un des alésages (5, 6) ou des alésages de plaque (11) présente une paroi intérieure en un matériau qui possède une dureté HP, et **en ce qu'**un insert en forme de cylindre creux ou de cône creux est logé dans l'alésage de plaque, lequel est au moins partiellement en contact avec la paroi intérieure et est adapté pour recevoir la tête d'une vis à os, l'insert étant disposé dans l'alésage de plaque de manière à ne pas pouvoir tourner et étant constitué d'un matériau qui présente une dureté HE < HP.

7. Plaque osseuse (1) selon l'une des revendications 1 à 6, **caractérisée en ce que** la partie distale de la tige (2) présente un trou de suture (10) de diamètre réduit par rapport au diamètre des trous (5, 6) et qui est adapté pour fixer les parties molles environnantes à la plaque osseuse (1).

8. Plaque osseuse (1) selon l'une des revendications 1 à 7, **caractérisée en ce que** l'épaisseur moyenne de la plaque osseuse (1) dans la zone de transition entre la partie de tige distale (2) et la partie de tête proximale (4) est inférieure à l'épaisseur moyenne de la partie de tige distale (2), de préférence inférieure de 10 %.

9. Plaque osseuse (1) selon l'une quelconque des revendications 1 à 8, **caractérisée en ce qu'**un rétrécissement (12) est prévu entre au moins deux trous de plaque (11) de la partie de tige distale (2), de sorte que la largeur de la partie de tige distale (2) est de préférence réduite d'au moins 20 %.

10. Plaque pour os (1) selon l'une des revendications 1 à 9, **caractérisée en ce que** la partie de tête proximale (4) présente un trou traversant (13) destiné à recevoir un fil de Kirschner, dont l'axe du trou est de préférence parallèle à l'un des axes (7, 8, 15) des trous (5, 6, 14).

11. Plaque osseuse (1) selon l'une des revendications 1 à 10, **caractérisée en ce que** la partie distale de la tige (2) est dépourvue d'ailettes latérales s'étendant transversalement à l'axe central (3), notamment celles comportant un trou de vis.

12. Plaque osseuse (1) selon l'une des revendications 1 à 11, **caractérisée en ce que** sa géométrie est adaptée à l'anatomie du cubitus droit (1).

13. Plaque osseuse (1) selon la revendication 12, **caractérisée en ce que** sa géométrie, inversée par rapport à celle-ci, est adaptée au cubitus gauche (1).

14. Combinaison d'une plaque osseuse (1) selon l'une des revendications 1 à 13 et d'au moins une vis à os polyaxiale.

15. Plaque osseuse (1) selon l'une des revendications 1 à 13, adaptée au traitement des fractures et des non-unions de l'olécrane et de l'ulna proximal ainsi que de ses ostéotomies.
